# EUROPÄISCHE PATENTSCHRIFT

(11) **EP 3 140 279 B1**
(45) Veröffentlichungstag und Bekanntmachung des Hinweises auf die Patenterteilung: **16.10.2019**
(21) Anmeldenummer: 15719240.2
(22) Anmeldetag: 05.05.2015
(51) Int. Cl.: B01J 31/02, C07C 68/02, C07C 68/08

(54) **VERFAHREN ZUR HERSTELLUNG VON DIARYLCARBONATEN**
PROCESS FOR PRODUCING DIARYLCARBONATES
PROCÉDÉ DE PRODUCTION DE DIARYLCARBONATES

(30) Priorität: 09.05.2014 EP 14167764
(43) Veröffentlichungstag der Anmeldung: 15.03.2017
(73) Patentinhaber: Covestro Deutschland AG, 51373 Leverkusen (DE)
(72) Erfinder: KÖHLER, Karl-Heinz, 52078 Aachen (DE); LEIBERICH, Ricarda, 63263 Neu-Isenburg (DE); HALLENBERGER, Kaspar, 51375 Leverkusen (DE); KRÄMER, Korbinian, 50733 Köln (DE); LIPSKI, Florian, 40882 Ratingen (DE); KAHNIS, Henning, 50667 Köln (DE); DENECKER, Gabriel, B-2920 Kalmthout (BE); VANDEN EYNDE, Johan, B-9052 Zwijnaarde (BE); SOMHOM, Weerachanan, Uttaradit 53000 (TH)
(74) Vertreter: Levpat
(86) Internationale Anmeldenummer: PCT/EP2015/059774
(87) Internationale Veröffentlichungsnummer: WO 2015/169775

(56) Entgegenhaltungen:
- EP-A1- 0 801 053
- WO-A1-2013/007601

## Beschreibung

Die Erfindung betrifft ein Verfahren zur Herstellung von Diarylcarbonaten aus Monophenolen und Phosgen oder Chlorameisensäurearylestern in Gegenwart von wenigstens einem ggf. substituierten Pyridin oder dessen Hydrochloridsalz als Katalysator, und dessen Rückgewinnung und Rückführung in das Verfahren. Das Verfahren wird zumindest teilweise in flüssiger Phase ohne Verwendung zusätzlicher Lösungsmittel durchgeführt, wobei der Katalysator über eine Kristallisation abgetrennt und zurückgewonnen wird.

Verfahren zur Herstellung von Diarylcarbonaten aus Monophenolen und Phosgen sind bekannt. Die Herstellung von Diarylcarbonaten (wie z. B. Diphenylcarbonat, "DPC") erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

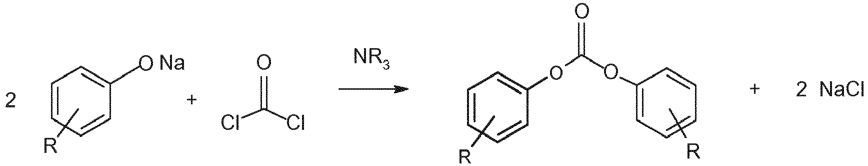

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

Dabei werden nach dem Phasengrenzflächen-Verfahren die in Lösemitteln und Wasser gelösten Edukte miteinander umgesetzt. Der Nachteil dieser Verfahren besteht in der destillativen Abtrennung des Diarylcarbonats vom Lösemittel und dessen Wiederaufarbeitung, sowie in der kochsalzhaltigen wässrigen Phase als Abfallprodukt, für die es nur begrenzte Verwendungsmöglichkeiten gibt und die gegebenenfalls sehr aufwendige Aufarbeitungsschritte erfordert.

Daher wurden Verfahren zur sogenannten Direkt-Phosgenierung von Monophenolen entwickelt, bei denen die Edukte Phosgen und Monophenol nicht in einem Phasengrenzflächenverfahren in Gegenwart von Alkalilauge, sondern in der Schmelze in Gegenwart von Katalysatoren, bevorzugt ohne Verwendung von zusätzlichen Lösemitteln, zu Diarylcarbonaten und Chlorwasserstoff, anstatt von Natriumchlorid, umgesetzt werden.

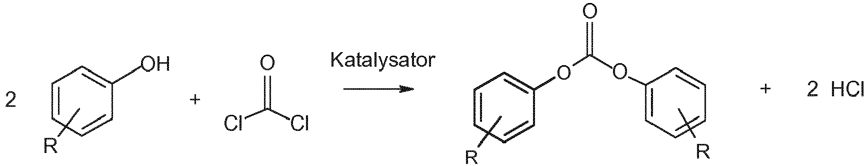

So wird beispielsweise in der US 2,362,865 (A) ein Verfahren zur Herstellung von Diarylcarbonaten durch Direkt-Phosgenierung von Monophenolen bei Temperaturen von 170°C bis 250°C unter Verwendung von A1- oder Ti-Phenolaten beschrieben, wobei aber keine Rückführung des Katalysators, geschweige denn eine Abtrennungsmethode beschrieben ist.

Sowohl in der EP 2 371 806 A1 als auch in der EP 2 371 807 A1 werden ebenfalls Verfahren zur Herstellung von Diarylcarbonaten durch Direkt-Phosgenierung von Monophenolen bei Temperaturen von 20°C bis 240°C unter Verwendung von Metall-Halogeniden oder Metall-Phenolaten beschrieben. Eine Rückführung des Katalysators in den Prozess ist ebenfalls nicht beschrieben worden.

In der EP 1 234 845 A wird ebenfalls die Umsetzung von Monophenolen in der Schmelze bei Temperaturen von 120°C bis 190°C mit einem besonders reinen Phosgen beschrieben. Als Katalysatoren werden Stickstoff-haltige Verbindungen, wie z.B. Pyridin in Mengen von 0,1 bis 10 mol% bezogen auf eingesetztes Monophenol verwendet. Auch diese Veröffentlichung gibt keine Hinweise auf eine Rückführung von Katalysator in den Prozess. Pyridin bildet mit Chlorwasserstoff ein schwerflüchtiges Salz (Siedepunkt: 222-224 °C), welches nicht ohne weiteres abdestilliert werden kann. Daher wird die Reaktionsmischung gemäß der Lehre von EP 1 234 845 A zunächst mit Natronlauge neutralisiert, so dass ein Gemisch aus Wasser, freiem Pyridin und überschüssigen Phenol abdestilliert werden kann.

Darüber hinaus gibt es eine Anzahl weiterer Schutzrechte, wie z.B. WO 2008/114750 A1, JP 2008-231073 A, JP 2009-114195 A, JP 09-278714 A, JP 09-100256 A, JP 10-245366 A, JP 11-012230 A in denen die Umsetzung von Monophenolen in der Schmelze mit Phosgen zu Diarylcarbonaten in Gegenwart homogen löslicher Stickstoff-haltiger Katalysatoren beschrieben wird.

In JP 10-077250 A, JP 09-24278 A und EP 1 234 845 A finden sich Hinweise auf eine mögliche Rückführung von Katalysator, ohne jedoch auf eine konkrete Abtrennung vom Katalysator vom Produkt sowie einer Aufarbeitungsmethode des Katalysators in Hinblick auf eine Rückführung einzugehen. Zudem wird auf eine Zufuhr von wässrigen Lösungen, insbesondere Wasser und/oder Natronlauge, im Zuge der Neutralisierung und Wäsche des Reaktionsgemisches hingewiesen.

US 5 239 106 lehrt die Abtrennung von Diphenylcarbonat aus katalysatorhaltigen Reaktionslösungen durch Kristallisation des 1:1 Addukts mit Phenol. Hier wird jedoch keine Katalysatorabtrennung und -rückführung beschrieben.

EP-A-0 801 053 offenbart die Rückgewinnung von Katalysatorsystemen aus Diarylcarbonat-haltigen Reaktionslösungen durch Suspensionskristallisation.

In keiner dieser Veröffentlichungen gibt es zufriedenstellende Hinweise auf Methoden zur Rückführung des Katalysators, wie z.B. Pyridin, in den Prozess. Insbesondere erfolgt nach einem Neutralisationsschritt mit wässriger, alkalischer Lösung die Abtrennung des Katalysators über die wässrige Phase.

Insbesondere gibt es im Stand der Technik keine konkreten Beispiele für ein Verfahren zur Abtrennung des Katalysators vom produkthaltigen Strom, bei dem nicht wie in den oben zitierten Dokumenten eine Neutralisation des Hydrochlorides mit wasserhaltigen Zusätzen erfolgt, die mit den oben beschriebenen Nachteilen behaftet ist.

In keiner dieser Veröffentlichungen ist ein komplett wasser- und abwasserfreies Verfahren zur Herstellung von Diarylcarbonaten dargestellt.

Die aus dem Stand der Technik bekannten Verfahren reichen daher nicht, um den hohen ökonomischen und ökologischen Anforderungen in Bezug auf Katalysator-Rückführung gerecht zu werden und darüber hinaus hohe Reinheiten der Produkte sicherzustellen, die ihrerseits Einsatzstoffe für weitere chemische Prozesse sind.

Für ein technisches Verfahren müssen jedoch wirtschaftliche Aspekte berücksichtigt werden. Die Rückführung des Katalysators gehört dabei zu den wichtigen Aspekten, die an dieser Stelle bewertet werden, denn eine hohe, oder gar komplette Ausschleusung des Katalysators bedeutet einen wirtschaftlicher Schaden und führt zu einer unerwünschten Umweltbelastung. Die entstehenden Abwässer müssen unter sehr hohem Aufwand gereinigt werden, was eine große Herausforderung für die Klärwerke bedeutet. In den Verfahren nach Stand der Technik ist entweder ein großer technischer Aufwand nötig, um eine Katalysatorrückführung zu ermöglichen, oder eine teilweise oder komplette Ausschleusung des Katalysators vorgesehen. In beiden Fällen fällt ein zusätzlicher Abwasserstrom an.

In einem Direktphosgenierungsverfahren ist die Bereitstellung einer effizienten Rückführung des Katalysators von höchster Bedeutung. Ferner sollte der Einsatz von wässrigen Lösungen, nicht nur während der Reaktion sondern auch bei der Aufarbeitung, möglichst vermieden werden. Denn organische Substanzen enthaltene Abwässer müssen zunächst aufwendig gereinigt und dann entsorgt werden.

Daher stellte sich die technische Aufgabe ein Verfahren zur Herstellung von Diarylcarbonaten nach dem Direkt-Phosgenierungs-Verfahren in der Schmelze eines Monophenols unter Verzicht auf zusätzliche Einsatzstoffe, wie Natronlauge und Wasser, zu entwickeln, das durch Reduzierung von Ausschleusen von Strömen (Purge) ökonomisch betrieben wird und bei gleichbleibend guter Qualität der Endprodukte, bereitgestellt.

Es wurde nun herausgefunden, dass bei Einsatz eines gegebenenfalls substituierten Pyridins oder eines seiner Salze als Katalysator, das entstehende Hydrochloridsalz, der Chlorwasserstoff und das Diphenylcarbonat durch eine Kombination von Destillation und Suspensionskristallisation voneinander zu trennen sind und dabei ein Diarylcarbonat von hoher Reinheit erzielt werden kann. Bevorzugt wird auf eine Neutralisation der Reaktionslösung bzw. Mutterlauge und / oder Zugabe von Wasser verzichtet. Dies führt zu einer besonders ökonomischen und ökologischen Verfahrensftihrung.

Gegenstand der Erfindung ist daher ein Verfahren zur Herstellung von Diarylcarbonat, vorzugsweise Diphenylcarbonat durch Reaktion eines Monophenols mit Phosgen und/oder wenigstens einem Chlorameisensäurearylester in Gegenwart wenigstens eines gegebenenfalls substituierten Pyridins, in freier Form und/oder in Form seines Hydrochloridsalzes, als Katalysator, wobei
a) die Reaktion in einem Reaktor unter Drücken von 1-50 bar (absolut) durchgeführt wird,
b) das Reaktionsgemisch aus dem Reaktor in einen zur Suspensionskristallisation geeigneten Apparat überführt wird,
c) in dem Apparat durch Temperaturabsenkung die Suspensionskristallisation eingeleitet wird,
d) das entstandene Kristallisat von der zurückbleibenden katalysatorhaltigen Mutterlauge getrennt wird,
e) die katalysatorhaltige Mutterlauge in den Reaktor des Schritts a) zumindest teilweise zurückgeführt wird.

Vorzugsweise wird in keinem der Schritte a) bis e) eine wässrige Lösung eingesetzt.

Die Reaktion in Schritt a) wird bevorzugt bei Temperaturen oberhalb von 80°C durchgeführt, um Abscheidungen von gebildeten Diarylcarbonaten in fester Form zu vermeiden. Die Umsetzung der Edukte kann sowohl bei Normaldruck oder leicht vermindertem Druck als auch bei erhöhten Drücken von bis zu 50 bar (absolut) durchgeführt werden. Hierbei kann, abhängig von den Prozessbedingungen, das Phosgen in kondensierter Phase vorliegen oder in der flüssigen Phase gelöst sein. Die nach diesem Verfahren hergestellten Diarylcarbonate sind aufgrund ihrer hohen Reinheit besonders geeignet für die Herstellung von hochreinen Polycarbonaten nach dem Schmelze-Umesterungsverfahren aus Diarylcarbonaten und Bisphenolen.

Der bei der Reaktion gewonnene Chlorwasserstoff kann einem oder mehreren Reinigungsschritten unterzogen werden, sodass er für eine Vielzahl weiterer Verwendungsmöglichkeiten geeignet ist, insbesondere für elektrochemische oder thermische Oxidationen zu Chlor. Dieses so gewonnene Chlor kann mit Kohlenmonoxid zur Phosgenherstellung verwendet werden; das erhaltene Phosgen kann im erfindungsgemäßen Verfahren eingesetzt werden.

Das unter den Reaktionsbedingungen flüssige Endprodukt wird in mehreren Trennschritten umfassend die Schritte b) bis d) von Nebenprodukten und dem Katalysator bzw. seinem HC1-Addukt abgetrennt. Danach weist es vorzugsweise einen Gehalt von mehr als 95%, bevorzugt mehr als 99,0%, besonders bevorzugt mehr als 99,5% Diarylcarbonat und ggf. Phenol auf. Bevorzugt enthält das Endprodukt mehrheitlich Diarylcarbonat. Der in der Reaktion verwendete Katalysator wird dabei so aufgearbeitet, dass er zumindest teilweise in die Reaktion zurückgeführt werden kann (Schritt e)).

Das erfindungsgemäße Verfahren besteht aus den drei Verfahrens-Abschnitten:
I. Reaktion umfassend den Verfahrensschritt a),
II. Chlorwasserstoff-Aufarbeitung (optional),
III. Produktreinigung und Katalysatorabtrennung durch Suspensionskristallisation umfassend die Verfahrensschritte b) bis d), wobei der rückgewonnene Katalysator zumindest teilweise in den Abschnitt I. zurückgeführt wird (Schritt e)).

In dem Verfahrens-Abschnitt I), Reaktion, werden die Edukte in einem vorgelagerten VerfahrensSchritt in der Weise miteinander vermischt, dass eine weitgehend homogene Lösung von Phosgen im geschmolzenen Monophenol vorliegt; dies kann gegebenenfalls durch Anwendung erhöhter Drücke bei den vorgegebenen Schmelzetemperaturen erfolgen.

Im Rahmen der Erfindung hergestellte Diarylcarbonate sind bevorzugt solche der allgemeinen Formel (I) 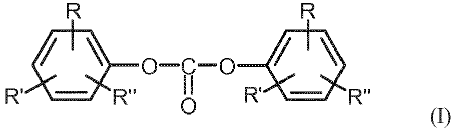 worin R, R' und R" unabhängig voneinander Wasserstoff, Halogen oder ein verzweigter oder unverzweigter C₁- bis C₉-Alkylrest oder ein verzweigter oder unverzweigter C₁- bis C₉-Alkoxycarbonylrest sein können. Bevorzugt sind R, R' und R" auf beiden Seiten der Formel (I) gleich.

Besonders bevorzugt ist Diphenylcarbonat.

Im Rahmen der Erfindung geeignete Monophenol(e) sind bevorzugt solche der allgemeinen Formel (II) 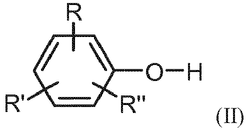 worin R, R' und R" unabhängig voneinander die für die allgemeine Formel (I) genannte Bedeutung haben können.

"C₁-C₄-Alkyl" steht im Rahmen der Erfindung beispielsweise für Methyl, Ethyl, n-Propyl, isoPropyl, n-Butyl, sec.-Butyl, tert.-Butyl; "C₁-C₆-Alkyl" darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl; "C₁-C₉-Alkyl" darüber hinaus beispielsweise für n-Heptyl und n-Octyl oder n-Nonyl. Gleiches gilt für den entsprechenden Alkylrest in Alkylcarbonylresten.

Geeignete Monophenole sind beispielsweise: Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol, 2,4,6-Tribromphenol, Anisol und Salicylsäure-methyl oder -phenylester.

Besonders bevorzugt ist Phenol.

Die eingesetzten Monophenole sollten eine Reinheit von mindestens 99,90 Gew% aufweisen.

Bevorzugt enthalten die Edukte weniger als 300 vol. ppm Wasser da die Anwesenheit von Wasser die Korrosion der Apparate-Materialien begünstigt.

Das hier eingesetzte Monophenol kann neben dem von außen in den Gesamtprozess eingeführte Phenol, dem sogenannten Frischphenol aus Vorratstanks, auch rückgeführtes Monophenol aus Kondensatströmen der Verfahrensschritte II) und III) oder aus Wasch-Flüssigkeitsströmen des Verfahrensschritts II) enthalten. Derartig rückgeführtes Monophenol kann Nebenprodukte aus dem Verfahren enthalten, wie z.B. Restmengen an Diarylcarbonat, Chlorwasserstoff oder Arylchlorkohlensäureester, die für die Reaktion unschädlich sind. In der eingesetzten Mischung der Edukte liegt das Monophenol vorzugsweise in mehr als der stöchiometrisch erforderlichen Menge bezogen auf Phosgen vor. Das molare Verhältnis von Monophenol zu Phosgen kann von 1,5:1 bis 4:1 variieren, bevorzugt ist ein Molverhältnis von 2:1 bis 3:1, besonders bevorzugt ist ein Molverhältnis von 2,5:1 bis 3:1.

Der Begriff "Chlorameisensäurearylester" wird im Folgenden für Verbindungen benutzt, welche bei der Herstellung von Diarylcarbonaten aus Monophenolen und Phosgen als Zwischenprodukt entstehen.

Im Rahmen der Erfindung geeignete Chlorameisensäurearylester sind bevorzugt solche der allgemeinen Formel (III) 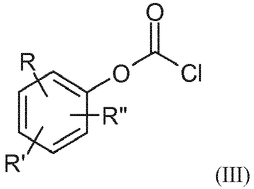 worin R, R' und R" unabhängig voneinander die für die allgemeine Formel (I) genannte Bedeutung haben können.
Soweit ein Chlorameisensäurearylester der allgemeinen Formel (III) mit einem Monophenol der allgemeinen Formel (11) umgesetzt wird, haben vorzugsweise R, R' und R" jeweils in den Formeln (I) und (11) dieselbe Bedeutung.

Besonders bevorzugt ist Chlorameisensäurephenylester.

Das eingesetzte Phosgen sollte zur Vermeidung von unerwünschten Nebenprodukten in den Endprodukten des Herstell-Verfahrens eine Reinheit von wenigstens 99,80 Gew%, vorzugsweise von 99,96 Gew% aufweisen; der Gehalt an Tetrachlorkohlenstoff sollte kleiner als 50 vol. ppm, vorzugsweise kleiner als 15 vol. ppm sein.

Erfindungsgemäß wird ein substituiertes oder unsubstituiertes Pyridin als Katalysator eingesetzt. Dieses kann in Form der freien Base oder ganz oder teilweise in Form seines Hydrochlorids vorliegen. "In Form der freien Base" bzw. "in freier Form" bedeutet im Sinne der Erfindung, dass der Stickstoff des Pyridinrings nicht protoniert vorliegt.

Vorzugsweise liegen höchstens 10 mol%, besonders bevorzugt höchstens 1 mol%, des gegebenenfalls substituierten Pyridins in freier Form vor. Der übrige Teil liegt in Form des Hydrochlorids vor.

Die erfindungsgemäß als Katalysator dienenden Pyridine sind vorzugsweise solche der allgemeinen Formel (IV) 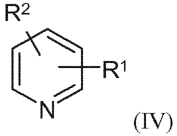 worin R¹ und R² unabhängig voneinander H, verzweigtes oder unverzweigtes C₁- bis C₉-Alkyl, C₅- oder C₆-Cycloalkyl, OH, OR³, NHR³ oder NR³R⁴ sein können, wobei R³ und R⁴ unabhängig voneinander für C₁- bis C₄-Alkyl stehen. Besonders bevorzugt stehen R¹ und R² für H.

Geeignete Pyridine sind beispielsweise Pyridin, 2-Picolin, 3-Picolin, 4-Picolin, 2-Ethylpyridin, 3-Ethylpyridin, 4-Ethylpyridin, 2-Isopropylpyridin, 3-Isopropylpyridin, 4-Isopropylpyridin, 2-Butylpyridin, 4-tert-Butylpyridin, 2,3-Lutidin, 2,4-Lutidin, 2,5-Lutidin, 2,6-Lutidin, 3,4-Lutidin, 3,5-Lutidin, 3,4-Diethylpyridin, 3,5-Diethylpyridin, 3-Ethyl-4-methylpyridin, 2-(3-Pentyl)pyridin, 4-(3-Pentyl)pyridin, 2-Dimethylaminopyridin, 4-Dimethylaminopyridin, 2-Methoxypyridin, 2,6-Dimethoxypyridin, 4-Cyclohexylpyridin, 4-(5-Nonyl)pyridin, 4-Phenylpropylpyridin und 2-Hydroxypyridin.

Besonders bevorzugt ist Pyridin.
In einer besonders bevorzugten Ausführungsform ist der Katalysator Pyridinhydrochlorid.

Die erfindungsgemäß zu verwendenden Katalysatoren können in Mengen von 0,001 mol% bis 10 mol%, vorzugsweise in Mengen von 0,01 mol% bis 5 mol%, bezogen auf vorhandenes Monophenol, eingesetzt werden.

Die Katalysatoren werden als Lösung in der Monophenol-Schmelze eingesetzt. Solche Lösungen enthalten erfindungsgemäß zumindest teilweise Katalysatormengen, die aus dem Verfahrensabschnitt III), mit oder ohne gesonderte Katalysator-Aufarbeitung, in die Reaktion als Verfahrensabschnitt I) zurückgeführt werden. Eine Katalysator-Aufarbeitung ist daher für die Rückführung des Katalysators in den Verfahrensabschnitt I) nicht zwangsweise erforderlich aber durchaus möglich.

Die Zugabe der Katalysatoren erfolgt frühestens nach erfolgter vollständiger Durchmischung der Edukte, vorzugsweise in dem Reaktor, um eine vorzeitige Reaktion der Edukte beim Vermischen und damit eine vorzeitige Chlorwasserstoff-Entwicklung in einem ungeeigneten Verfahrensabschnitt zu vermeiden.

Eine Rückführung von Katalysatormengen aus dem Verfahrensabschnitt III) kann beliebig häufig vorgenommen werden; bei kontinuierlichen Verfahren kann vorzugsweise eine Teilmenge des Katalysators kontinuierlich zurückgeführt werden, während ggf. eine Teilmenge aus dem Verfahrens-Kreislauf ausgeschleust wird, um Verunreinigungen des Katalysators vorzubeugen oder ggf. einer Desaktivierung des Katalysators Rechnung zu tragen. Bei Bedarf kann frischer Katalysator der zurückgeführten Katalysatormenge hinzugefügt werden. In einer bevorzugten Ausführungsform werden wenigstens 25 Gew.% des Katalysators zurückgeführt, besonders bevorzugt wenigstens 50 Gew.%, ganz besonders bevorzugt wenigstens 75 Gew.% und insbesondere ganz besonders bevorzugt wenigstens 85 Gew.%. In einer bevorzugten Ausführungsform werden jedoch maximal 99 Gew.% des Katalsyators zurückgeführt, bevorzugt maximal 95 Gew.%.

Die Edukte Monophenol und Phosgen werden in den oben angegebenen molaren Verhältnissen bzw. in den oben genannten bevorzugten molaren Verhältnissen miteinander gemischt, wobei das Monophenol stets als Schmelze vorliegt und das Phosgen je nach vorherrschendem Druck gasförmig oder flüssig ist. Bei Normaldruck und Temperaturen oberhalb von 60°C liegt weitgehend ein zweiphasiges Gas-Flüssigkeitsgemisch vor, da die Löslichkeit von Phosgen auch in Monophenolen, ebenso wie in Diarylcarbonaten bei zunehmender Temperatur abnimmt.

Daher müssen Gemische aus geschmolzenen Monophenolen und Phosgen in der Reaktionsphase sehr intensiv gemischt und redispergiert werden, um über eine ausreichende Erneuerung der Phasengrenzflächen einen hinreichenden Umsatz der Edukte zu gewährleisten. Alternativ kann der Umsatz von Phosgen mit Phenol in einer kondensierten homogenen Phase (aufgrund der im Vergleich zum zweiphasigen Gemisch von gasförmigem Phosgen und flüssigem Phenol erhöhten Konzentration vom Phosgen im Phenol) deutlich erhöht werden,. Auch die Temperaturerhöhung wirkt beschleunigend auf die Reaktionsgeschwindigkeit, so dass erhöhte Temperaturen im Bereich von 100°C bis 250°C, vorzugsweise 110°C bis 220°C von Vorteil sein können. Da solche Temperaturen, wie oben genannt, allerdings der Löslichkeit von Phosgen in Phenol entgegen wirken, ist eine Reaktionsführung bei erhöhter Temperatur unter Druck besonders vorteilhaft. Deshalb werden die Edukte bei erhöhter Temperatur unter Normaldruck, vorzugsweise unter erhöhtem Druck bis zu 50 bar (absolut), besonders bevorzugt bei erhöhtem Druck bis 30 bar (absolut) und ganz besonders bevorzugt bei Drücken von 4 bis 25 bar (absolut) miteinander vermischt und zur Reaktion gebracht. Die Temperatur in der Mischzone sollte mindestens die Schmelztemperatur des Monophenols betragen, vorteilhaft ist aber eine Reaktionstemperatur im Bereich von 100°C bis 250°C.

Nach der weitgehend vollendeten Mischung der Edukte wird dem Gemisch vorzugsweise einer der oben genannten Katalysatoren vorzugsweise in der bevorzugten Menge als Lösung im Monophenol hinzugesetzt. Da bei den oben genannten Temperaturen und Drücken die katalysierte Umsetzung von Monophenol mit Phosgen zum Arylchlorkohlensäureester als Zwischenprodukt sehr rasch unter Abspaltung von gasförmigem Chlorwasserstoff verläuft, kann die Reaktion bevorzugt mehrstufig durchgeführt werden. Die Reaktion kann unter adiabatischen Bedingungen geführt werden, da sie nur eine leichte Wärmetönung aufweist. In einer ersten Stufe, dem sogenannten Hauptreaktor, entstehen insbesondere bei erhöhtem Druck und bevorzugt bei Temperaturen von 120°C bis 230°C, besonders bevorzugt bei Temperaturen von 130°C bis 210°C und für die Herstellung von Diphenylcarbonat ganz besonders bevorzugt bei einer Temperatur von 170°C bis 200°C und bei Reaktor-Flüssigkeitsverweilzeit von 15 bis 120 min , vorzugsweise von 45 bis 90 min, überwiegend Arylchlorkohlensäureester neben bereits weiterreagiertem Diarylcarbonat. In einer zweiten Stufe reagiert in einem sogenannten Nachreaktor der Arylchlorkohlensäureester bei etwas höheren Temperaturen von vorzugsweise 170°C bis 250°C, besonders bevorzugt von 190°C bis 230°C und ganz besonders bevorzugt von 200°C bis 210°C bei Reaktor-Verweilzeiten von 15 bis 120 min, vorzugsweise von 45 bis 90 min mit noch vorhandenem Monophenol zum Diarylcarbonat. Dabei kann der Druck in der zweiten Stufe im sogenannten Nachreaktor auch auf 2 bis 20 bar abgesenkt werden. Eine derartige Absenkung des Druckes kann vorteilhaft in einer sogenannten Flash-Stufe vorgenommen werden, in der als Folge der Druck-Absenkung das im Hauptreaktor entstandene Chlorwasserstoff-Gas besonders gut aus der Reaktions-Schmelze abgetrennt werden kann. Auch hinter der zweiten Reaktions-Stufe im Nachreaktor kann sich optional eine Flash-Stufe zur Abtrennung der Restmengen an Chlorwasserstoff befinden.

Als Reaktoren für die Umsetzung der Edukte unter den angegebenen Reaktions-Bedingungen sind vorzugsweise kontinuierliche Reaktoren gut geeignet, möglich ist aber auch die Verwendung von Rührkesseln als batch-Reaktoren. Besonders gut geeignete kontinuierliche Reaktoren sind z.B. Rührkesselkaskaden, Blasensäulen-Kolonnen, Boden-Kolonnen, Füllkörper-Kolonnen oder Kolonnen mit feststehenden Einbauten zur Durchmischung des Reaktionsmediums oder Reaktions-Destillations-Kolonnen.

Solche Kolonnen können auch mit einander kombiniert sein, wie z.B. eine Blasensäulen-Kolonne mit einer aufgesetzten Rektifikations-Kolonne, wobei abweichend von der oben beschriebenen Mischung der Edukte, die Edukte getrennt an unterschiedlichen Stellen der Kolonnen-Kombination eingeführt werden können. So kann z.B. in der oben genannten Kolonnen-Kombination das Phosgen in die untere Blasensäulen-Kolonne und das Monophenol zusammen mit dem Katalysator in die obere Rektifikations-Kolonne mit ca. 10 theoretischen Böden eingeführt werden. Das entstandene Diarylcarbonat wird aus der Blasensäulenkolonne entnommen.

Eine entsprechend getrennte Dosierung der Edukte kann auch in einer Reaktions-DestillationsKolonne derart erfolgen, dass das Phosgen in der Mitte der Kolonne eingeführt wird und dass das Monophenol zusammen mit Katalysator am Kopf der Kolonne eingebracht wird. Das Reaktionsgemisch wird dem Kolonnen-Sumpf entnommen. Solche Kolonnen können mindestens 5, vorzugsweise ca. 20 Böden aufweisen.

In einer weiteren optionalen Ausführungsform der Reaktoren können die Edukte in einem Hauptreaktor bei Drücken von 1 bis 25 bar (absolut) bei ausreichend hoher, gegebenenfalls längerer Verweilzeit aber geringeren Temperaturen im unteren Teil des Reaktors von vorzugsweise 120°C bis 190°C, besonders bevorzugt von 160°C bis 180°C vollständig umgesetzt werden. Im oberen Teil des Reaktors ist eine zusätzliche Beheizung erforderlich, um dort etwas höhere Temperaturen bis 250°C, vorzugsweise bis 230°C zu realisieren. Die weitgehende Entgasung des Reaktionsgemischs und Abtrennung der Leichtsieder kann anschließend durch eine Flash-Verdampfung oder eine andere Entgasungstechnik erfolgen.

Besonders bevorzugt sind Blasensäulen-Kolonnen, die mit dem Edukt-Gemisch wie oben beschrieben von unten nach oben durchströmt werden. Dabei werden am Kolonnen-Kopf der Blasensäulen-Kolonne der gasförmige Chlorwasserstoff und am oberen Ende des Kolonnen-Schafts das Reaktionsgemisch entnommen. Dieses wird der nächsten Blasensäulen-Kolonne, die als Nachreaktor fungiert, über den Kolonnen-Boden zugeführt. Aus der letzten Blasensäulen-Kolonne wird das vollständig ausreagierte Reaktionsgemisch am Ende eines Verweil-Reaktors entnommen und dem nachfolgenden Verfahrensabschnitt III), der Produktreinigung und Katalysatorabtrennung durch Suspensionskristallisation, zugeführt. Das jeweils am Kopf der Blasensäulen-Kolonnen entnommene Chlorwasserstoff-Gas wird in dem nachfolgenden Verfahrensabschnitt II), der Chlorwasserstoff-Aufarbeitung vereinigt. Eine zusätzliche Abtrennung von Chlorwasserstoff ist auch zwischen den einzelnen Stufen durch Entspannen in einem Flash und eine anschließende Druckerhöhung möglich.

Die Apparate-Materialien müssen den hohen Anforderungen an Beständigkeit gegenüber Chlorwasserstoff und Phosgen bei hohen Temperaturen entsprechen und werden vorzugsweise ausgewählt aus der Gruppe der Werkstoffe Schwarzer Stahl, Edelstahl, Stahl-Legierungen, Nickel-Basislegierungen (z.B. Hastelloy C), Keramik, Graphit, Email-beschichtete Materialien, PTFE-verkleidete Materialien.

Ziel des optionalen Verfahrensabschnitts II), der Chlorwasserstoff-Aufarbeitung, ist die Abtrennung und Aufreinigung des Nebenprodukts Chlorwasserstoff. Dazu wird die in der Reaktion A) entstandene Gasphase gesammelt und das Chlorwasserstoff-Gas von den übrigen Komponenten getrennt, die gegebenenfalls für eine weitere Umsetzung zu Diarylcarbonat zurückgeführt werden können. Das Nebenprodukt Chlorwasserstoff kann zur Erhöhung der Reinheit destilliert werden. Ferner kann der gasförmige Teilstrom aus Verfahrensabschnitt III) beigemischt werden.

In diesem Verfahrensabschnitt II) werden die HCl-haltigen Stoffströme aus Verfahrensabschnitt I) vereinigt und gemeinsam gereinigt. Vorzugsweise wird der Chlorwasserstoff dabei nicht neutralisiert. Hauptprodukt unter den leichtsiedenden Komponenten ist mit 94 Gew% oder mehr das Chlorwasserstoff-Gas; Nebenprodukte sind das im Überschuss eingesetzte Monophenol mit mehr als 3 Gew %, sowie Spuren an Arylchlorkohlensäureester, Diarylcarbonat und Phosgen und als Nebenprodukt aus dem Phosgen Spuren an Kohlenmonoxid und Tetrachlorkohlenstoff. Die Nebenprodukte können über verschiedene Schritte vom Hauptprodukt Chlorwasserstoff weitgehend abgetrennt werden, sodass ein Chlorwasserstoff-Gas mit einer Reinheit von mehr als 99,0 Vol.%, vorzugsweise von mehr als 99,8 Vol.% und einem Restgehalt an Phosgen und/oder Chlor-Kohlensäureestern von weniger als 1000 vol. ppm, vorzugsweise von weniger als 500 vol. ppm erhalten wird. Ebenso sollte der Gehalt an organischen Verbindungen im Chlorwasserstoff kleiner als 1000 vol. ppm, vorzugsweise kleiner als 50 vol. ppm sein, insbesondere der Gehalt an chlorhaltigen Kohlenwasserstoffen sollte kleiner 50 vol. ppm sein.

Diese Aufgabe wird durch einen oder mehrere Schritte gelöst, die nachfolgend beschrieben sind. Vorzugsweise wird diese Aufgabe durch einen mehrstufigen Prozess gelöst. Vorzugsweise erfolgt die Abtrennung des Chlorwasserstoffs destillativ.
In einer sogenannten ersten Kondensations-Stufe werden die Nebenprodukte mit einem höheren Siedepunkt als dem von Chlorwasserstoff bei geeigneter Temperatur auskondensiert. Dabei werden insbesondere höher siedende Komponenten, die in größerer Konzentration vorliegen, wie Monophenole und Diarylcarbonate weitgehend aus dem Chlorwasserstoff-Gas entfernt, die in die Reaktion zurückgeführt werden können. Diese Abtrennung gelingt besonders gut, wenn neben der tieferen Temperatur optional auch erhöhte Drücke angewendet werden. Bevorzugte Temperaturen in der ersten Kondensationsstufe sind mindestens 80°C, und für die Herstellung von Diphenylcarbonat besonders bevorzugt 90°C. Der Druck wird vorzugsweise in einem Bereich von 8 bis 25 bar (absolut) eingestellt, ein besonders bevorzugter Druck für die Herstellung von Diphenylcarbonat ist 12 bar (absolut). Die Kondensation der Nebenprodukte aus dem Chlorwasserstoff-Gas-Strom kann optional auch mehrstufig bei verschiedenen Temperaturen und / oder Drücken erfolgen.

Falls eine ausreichend tiefe Temperatur oder ein ausreichend hoher Druck technisch nicht oder nur schwer zu realisieren ist, kann diese erste Kondensations-Stufe auch übergangen werden, um die Nebenprodukte in einer nachfolgenden sogenannten HCl-Wasch-Stufe in einer geeigneten Apparatur mit geschmolzenem Diarylcarbonat aus dem Chlorwasserstoff-Strom heraus zu waschen. Stellt diese HCl-Waschstufe die erste Reinigungsstufe für den Chlorwasserstoff unter Umgehung der ersten Kondensationsstufe dar, so kann diese HCl-Waschstufe auch in sich mehrstufig ausgebildet sein und auf verschiedenen, fallenden Temperatur-Niveaus betrieben werden, um die Effizienz der Wäsche zu erhöhen. Dabei lösen sich insbesondere Monophenole sehr gut im Diarylcarbonat. Auch Spuren an Chlorkohlensäureester und Phosgen können in diesem Verfahrensschritt noch zum Diarylcarbonat umgesetzt werden, wenn das zur Wäsche eingesetzte Diarylcarbonat z. B. an einer geeigneten Stelle im nachfolgenden Verfahrens-Abschnitt III), der Diarylcarbonat-Aufarbeitung entnommen wird. Prinzipiell ist jeder Diarylcarbonat-Strom dieses Verfahrens-Abschnitts bis hin zum destillierten Diarylcarbonat für die HCl-Wasch-Stufe geeignet, vorteilhaft für die Umsetzung der genannten organischen Chlor-Verbindungen kann es sein, einen Katalysator- und Phenol-haltigen Diarylcarbonat-Strom für die HCl-Wasch-Stufe dem Verfahrensabschnitt III) zu entnehmen, um in kurzer Zeit die im Chlorwasserstoff-Gas noch vorhandenen organischen Chlor-Verbindungen zur Reaktion bringen zu können.

Ein solches geeignetes Diarylcarbonat ist das Verfahrens-Abschnitts I) (Reaktion) verlassende Roh-Diarlycarbonat, das zur weiteren Aufarbeitung der ersten Stufe des Verfahrens-Abschnitts III) (Diarylcarbonat-Aufarbeitung) zugeleitet wird. In diesem Diarylcarbonat sind ausreichende Mengen an Katalysator und an Monophenol vorhanden. Alternativ dazu kann ein destilliertes Diarylcarbonat in beliebiger Weise für die HCl-Wasch-Stufe verwendet werden, da die physikalische Löslichkeit der auszuwaschenden Nebenprodukte im DPC hinreichend hoch ist. Bevorzugt jedoch wird ein reines destilliertes Diarylcarbonat für die HCl-Waschstufe verwendet. Zur Umsetzung der organischen Chlorverbindungen in der HCl-Wasch-Stufe kann anstelle des Diarylcarbonats als Waschmedium ebenso auch das Monophenol verwendet werden, da auch im Monophenol die physikalische Löslichkeit der auszuwaschenden Nebenprodukte hinreichend hoch ist. Dieses Monophenol kann z.B. ein Teilstrom des Monophenol-Edukt-Stromes sein. Falls eine Reaktion von Chlorestern oder Phosgen zu Diarylcarbonat erwünscht ist, so kann das zur Wäsche verwendete Monophenol in beliebiger Weise Katalysator enthalten. Die HCl-Wäsche mit Diarylcarbonat oder mit Monophenol wird vorzugsweise bei Temperaturen oberhalb des Schmelzpunktes des Diarylcarbonats durchgeführt; bei der Herstellung von Diphenylcarbonat wird eine Schmelzetemperatur von 80-95°C besonders bevorzugt. Die HCl-Wäsche kann bei Normaldruck oder bei erhöhtem Druck von 8 bis 25 bar (absolut) durchgeführt werden; bei der Herstellung von Diphenylcarbonat sind 12 bar (absolut) besonders bevorzugt.

Bei einer derartigen Wäsche kann ein Chlorwasserstoffgas mit einer Reinheit von mehr als 99,8 Gew% erhalten werden. Vorzugsweise liegt der Anteil an Phosgen unter 500 vol. ppm, der von Chlorameisensäureester unterhalb der Nachweisgrenze und der Phenolgehalt wird bis auf unter 10 vol. ppm verringert.

Diese HCl-Wasch-Stufe ist nicht zwingend erforderlich und kann bei beliebiger Kombination anderer Verfahrens-Schritte miteinander auch umgangen werden.

Zur Erzielung hoher Reinheiten des Chlorwasserstoff-Gases ist eine Chlorwasserstoff-Destillation besonders gut geeignet. Um eine solche Destillation energieeffizient durchführen zu können, ist die vorangehende Abkühlung des zu reinigenden Chlorwasserstoffs auf tiefere Temperaturen in einer vorgeschalteten zweiten Kondensations-Stufe sinnvoll, aber nicht zwingend erforderlich. Entfällt diese Stufe, dann ist in der nachfolgenden Chlorwasserstoff-Destillation eine entsprechend höhere Energie bei tiefen Temperaturen erforderlich. In dieser zweiten Kondensations-Stufe, die optional auch auf mehreren unterschiedlichen Temperatur- und /oder Druck-Niveaus arbeiten kann, werden die im Chlorwasserstoff-Gas noch enthaltenen Spuren an höher siedenden Nebenprodukten abgeschieden, insbesondere bei Anwendung höherer Drücke im Bereich von 8 bis 25 bar (absolut), bei Diphenylcarbonat bevorzugt 12 bar (absolut). Die Temperaturen können in Abhängigkeit von den technischen Gegebenheiten in einem sehr weiten Bereich von plus 25°C bis minus 50°C variieren. Diese zweite Kondensations-Stufe ist insbesondere dann sehr empfehlenswert, wenn die Wäsche in der HCl-Wasch-Stufe mit Monophenol durchgeführt wurde, da auf diese Weise die im HCl-Gas-Strom vorhandene Konzentration an Monophenol deutlich abgesenkt werden kann und damit die HCl-Destillation entlastet wird. Entfällt diese zweite Kondensations-Stufe, so sind die Anforderungen an den Energie-Bedarf in der HCl-Destillation entsprechend sehr viel höher. Die Kondensate können ebenfalls, wie in der ersten Kondensations-Stufe der Reaktion zugeführt werden.

Als vierte und letzte Stufe der Chlorwasserstoff-Aufarbeitung im Verfahrensabschnitt II) ist die Chlorwasserstoff-Destillation in einer besonders bevorzugten Ausführungsform besonders gut geeignet zur Herstellung eines hochreinen Chlorwasserstoffs. Sie sollte vorzugsweise bei erhöhtem Druck durchgeführt werden, da sonst der energetische Aufwand für das Einstellen alternativ erforderlicher hinreichend tiefer Temperaturen unverhältnismäßig hoch wäre. Sollten vorangegangene Reinigungs-Stufen bei Normal-Druck durchgeführt worden sein, so ist spätestens bei dieser Reinigungs-Stufe eine Verdichtung des Chlorwasserstoff-Stromes auf höhere Drücke von 8 bis 25 bar (absolut) sehr empfehlenswert; für die Herstellung von Diphenylcarbonat sind 12 bar (absolut) besonders bevorzugt. Unter diesen Bedingungen ist ein Chlorwasserstoffgas mit einer Reinheit von 99,95 Gew % erhältlich.

Alle der vier oben genannten Stufen der Chlorwasserstoff-Reinigung im Verfahrensabschnitt II) sind in der beschriebenen Reihenfolge erfindungsgemäß besonders gut zur Herstellung eines hochreinen Chlorwasserstoff-Gases geeignet. Die Einhaltung bestimmter Reihenfolgen oder die Durchführung aller Verfahrens-Stufen ist nicht zwingend erforderlich, sondern hängt ab von dem Grad der Verunreinigung des aus der Reaktion abgeschiedenen Chlorwasserstoffs und von dem gewünschten Reinheitsgrad des Chlorwasserstoff-Gases als Endprodukt. So ist es gegebenenfalls durchaus möglich, mit einzelnen Reinigungs-Stufen oder einer einzigen Reinigungs-Stufe das gewünschte Ergebnis zu erzielen, wie im Folgenden am Beispiel der HCl-Destillation dargestellt.

Werden die Eingangsstoff-Ströme aus dem Verfahrensabschnitt I) (Reaktion) ohne vorherige Reinigung direkt der Chlorwasserstoff-Destillation zugeführt, so ist unter den gleichen Temperatur- und Druck-Bedingungen ebenfalls ein Chlorwasserstoffgas mit einer Reinheit von 99,95 Gew% erhältlich.

Eine Kombination der Reinigungsstufen ist durchaus in einer bestimmten, jedoch von oben genannter Aufzählung unabhängigen Reihenfolge zur Erzielung bestimmter Reinheitsgrade ausführbar.

Als Apparate zur Durchführung der ersten und zweiten Kondensations-Stufe sind klassische Kühlfallen mit einer für die Prozess-Bedingungen ausreichend hohen Wärmetauscher-Oberfläche und einer Vorrichtung zur Ausspeisung der Kondensate in die Reaktion geeignet. Solche Kühlfallen können auch mehrstufig ausgeführt und gegebenenfalls verschieden temperiert sein. Geeignete Apparate für die HCl-Wasch-Stufe sind insbesondere kontinuierlich betriebene Apparate, wie z.B. Blasensäulen-Kolonnen, Glockenboden-Kolonnen, Füllkörper-Kolonnen, Packungskolonnen, Kolonnen mit feststehenden Einbauten, in denen die Waschflüssigkeit von oben dem aufsteigenden Chlorwasserstoff-Gas entgegen geführt werden. Auch kontinuierlich betriebene Rührapparate, wie z.B. Mixer-Settler oder auch diskontinuierlich betriebene Rührapparate sind prinzipiell geeignet.

Die Chlorwasserstoff-Destillation kann in üblichen Destillations- oder Rektifikations-Kolonnen mit geeigneten Kolonnen-Einbauten vorgenommen werden.

Die Materialien für die oben genannten Apparate müssen den hohen Anforderungen an Beständigkeit gegenüber Chlorwasserstoff bei hohen Temperaturen entsprechen und werden vorzugsweise ausgewählt aus der Gruppe Schwarzer Stahl, Edelstahl, Stahl-Legierungen, Nickel-Basislegierungen (z.B. Hastelloy C), Keramik, Graphit, Email-beschichtete Materialien, PTFEverkleidete Materialien.

Im Verfahrensabschnitt III), der Produktaufreinigung und Katalysatorabtrennung, werden die in der Reaktion I) entstandenen höher siedenden Komponenten gesammelt, getrennt und der Katalysator, in Form der freien Base oder in Form des Hydrochlordids, in die Reaktion zurückgeführt. Das Hauptprodukt wird dabei so weit gereinigt, dass ein Diarylcarbonat mit einer Reinheit von mehr als 99,0 Gew%, vorzugsweise von mehr als 99,8 Gew%, besonders bevorzugt mehr als 99,95 Gew.-% erhalten wird.

Es wurde herausgefunden, dass eine Isolierung und Rückführung des Katalysators aus dem Reaktionsgemisch mittels eines Suspensionskristallisationsschritts möglich ist. Bevorzugt wird ein thermischer Trennschritt vor der Kristallisation eingefügt, besonders bevorzugt eine Kombination eines destillativen Trennschritts mit einem Suspensionskristallisationsschritt. Mit Hilfe des destillativen Trennschritts kann das Verhältnis von Monophenol zu Diarylcarbonat für die nachfolgende Suspensionskristallisation (Schritt c) eingestellt werden.

Vorzugsweise beträgt das Verhältnis von Monophenol zu Diarylcarbonat in der in Schritt c) eingesetzten Lösung mindestens 0,5 : 1, besonders bevorzugt mindestens 1 : 1, ganz besonders bevorzugt mindestens 1,5 : 1.

In einer bevorzugten Ausführungsform beträgt das Verhältnis von Monophenol zu Diarylcarbonat in der in Schritt a) erhaltenen Lösung mindestens 0,5 : 1, besonders bevorzugt mindestens 1 : 1, ganz besonders bevorzugt mindestens 1,5 : 1.

In einer anderen bevorzugten Ausführungsform wird das Verhältnis von Monophenol zu Diarylcarbonat vor dem Schritt c) mit Hilfe eines thermischen Trennverfahrens auf mindestens 0,5 : 1, besonders bevorzugt mindestens 1 : 1, ganz besonders bevorzugt mindestens 1,5 : 1 eingestellt.

Erfindungsgemäß wird die Suspensionskristallisation in Schritt c) durch Temperaturabsenkung eingeleitet. Vorzugsweise wird die Temperatur dabei auf unter 70 °C, vorzugsweise unter 60°C abgesenkt.

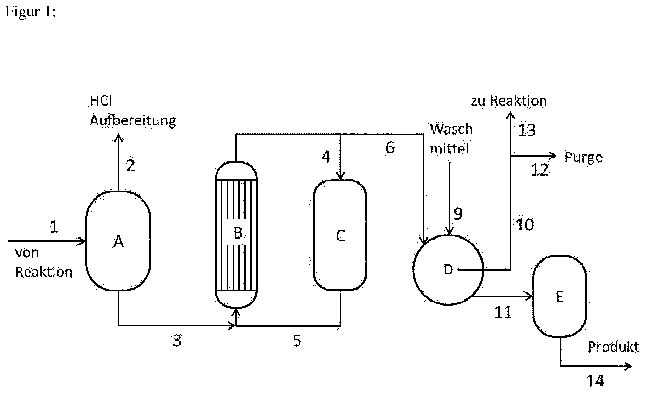

Eine graphische Übersicht dieses Verfahrens-Abschnitts gibt Figur 1.

In einem ersten Trennungsschritt des flüssigen Reaktionsgemisches wird in einer Entgasungsstufe gelöster Chlorwasserstoff weitestgehend abgetrennt. Dies kann mit einem Flash (A in Figur 1), einer Destillationskolonne, einer Kombination dieser Apparate oder einer weiteren üblichen Entgasungstechnik (z.B. Strippung) erreicht werden.

Bevorzugt ist der Einsatz einer Flashstufe (A), wobei durch Druckabsenkung eine Ausgasung des gelösten Chlorwasserstoffs erfolgt. Dabei werden Drücke von 20 mbar bis 1 bar (absolut) und Temperaturen von 140°-205°C gewählt, bevorzugt Drücke von 0,1 bar bis 1 bar (absolut) und Temperaturen von 165-205°C und besonders bevorzugt Drücke von 0,3-0,7 bar (absolut) und Temperaturen von 180-200°C.

Für die Abtrennung von Chlorwasserstoff kann alternativ eine Destillationskolonne eingesetzt werden, die bei Drücken von 200 mbar bis 2 bar (absolut), bevorzugt von 0,5 bar bis 1 bar (absolut), besonders bevorzugt von 0,8-1,0 bar (absolut) betrieben wird.

In der Dampfphase des Flashs bzw. am Kopf der Destillationskolonne wird ein Gemisch aus Chlorwasserstoff, Monophenol und freiem, ggf. substituierten Pyridin gewonnen. Dieses Gemisch wird bevorzugt in Verfahrensabschnitt II dem Hauptgasstrom zur Gasaufbereitung zugesetzt.

Der Sumpf des Flash bzw. der Kolonne ist weitestgehend frei von Chlorwasserstoff und in der bevorzugten Variante arm an Monophenol. Die Zusammensetzung des Sumpfes ergibt sich somit aus Diarylcarbonat, Monophenol, ggf. substituierten Pyridin, in freier Form und in Form des Hydrochlorids und Nebenprodukten.

In weiteren Varianten können Flashstufen und Destillationskolonnen zur Abtrennung von Chlorwasserstoff kombiniert werden oder eine weitere Entgasungstechnik eingesetzt werden (z.B. Strippung). Alternativ kann der erste Trennungsschritt auch ausgelassen werden. Dies ist aber nicht bevorzugt, weil sich so größere Prozessströme für den zweiten Trennungsschritt ergeben und der in die weiteren Trennungsstufen überführte Chlorwasserstoff Korrosionsprobleme hervorrufen kann.

Im zweiten Trennungsschritt wird der Katalysator aus der Diarylcarbonat-haltigen, von Chlorwasserstoff gereinigten Reaktionslösung durch Suspensionskristallisation zurückgewonnen, wobei man eine das Katalysatorsystem enthaltende Mutterlauge und ein Kristallisat erhält. Die Mutterlauge kann zumindest teilweise in den Reaktor zur Herstellung des Diarylcarbonats zurückgeführt oder weiter aufgearbeitet werden. Bevorzugt wird die Mutterlauge wenigstens teilweise aufbereitet und diese aufbereitete Mutterlauge wenigstens teilweise in den Reaktor zur Herstellung des Diarylcarbonats zurückgeführt. Das Kristallisat kann auf reines Diarylcarbonat und reines Monophenol aufgearbeitet werden

Beim erfindungsgemäßen Verfahren wird die Reaktionslösung aus dem Reaktionsteil I) entnommen und in einer Suspensionskristallisation des Reaktionsproduktes, gegebenenfalls durch Impfen der Reaktionslösung mit Diarylcarbonat-Monophenol-Addukt-Kristallen, eine katalysatorhaltige Mutterlauge gewonnen. Eventuelle Reste des Katalysators können aus dem Kristallisat mit einer wasserfreien Waschlösung, bevorzugt einem Gemisch aus Diarylcarbonat und aromatischer Hydroxyverbindung, gewaschen werden. Die so vom Katalysatorsystem und anderen Verunreinigungen befreiten Kristallisate, bestehend aus einem Gemisch aus Diarylcarbonat und aromatischer Hydroxyverbindung können verlustfrei durch Kristallisation oder Destillation in hochreines Diarylcarbonat aufarbeitet und die das Katalysatorsystem enthaltende Reaktionslösung in den Reaktor zurückführt werden. Die Waschlösung kann anschließend ohne weitere Behandlung der Reaktion als Feedergänzung der aromatischen Hydroxyverbindung zugeführt werden.

Überraschenderweise wurde im Falle des Diarylcarbonat/Monophenol-Systems gefunden, dass sich die Zusammensetzung der Kristallisate in Abhängigkeit vom Diarylcarbonatgehalt der Reaktionslösung ändert und nur in einem sehr engen Konzentrationsbereich das 1 : 1-Addukt auftrat.

Aus der Reaktionslösung wird vorzugsweise nur so viel Diarylcarbonat durch Kristallisation abgetrennt, wie durch die Reaktion neu entsteht und zur Waschung der Kristallisate notwendig ist.

Der Restgehalt an Diarylcarbonat wird gemeinsam mit den anderen Bestandteilen der Mutterlauge wie der dem Reaktor zugeführt. Eine thermische Schädigung des Katalysatorsystems findet nicht statt, was die Katalysatordesaktivierung auf ein Minimum reduziert.

Für das erfindungsgemäße Verfahren können Kristallisationstechnologien eingesetzt werden, wie sie beispielsweise in Chem.-Ing.-Techn. 57 (1985) 91 ff ausführlich beschrieben sind. Die häufig verwandten Rührkessel-Kristallisatoren, vergl. Chem.- Ing.-Techn. 57 (1985) S. 95, können nach Vorschriften und Empfehlungen von Ullmann's Encyclopedia of Industrial Chemistry, Vol B 2, Unit Operations I (1988), Chapter 25: Stirring, dimensioniert werden. Diese Verfahren werden diskontinuierlich oder kontinuierlich durchgeführt. Alle Apparate haben Wärmeaustauschflächen und einen Kühlmittelkreislauf; die unten angegebenen Temperaturen sind die des von den Wärmeaustauschflächen zurücklaufenden Kühlmittels.

Figur 1 zeigt eine besonders bevorzugte Ausführungsform des erfindungsgemäßen Verfahrens. Die Buchstaben in Figur 1 haben folgende Bedeutungen:
A: Flashstufe
B: Umlaufkühler
C: Kristaller
D: Trommeldrehfilter
E: Aufschmelzer

Die Ziffern 1-14 haben die folgenden Bedeutungen:
1: Reaktionsgemisch
2: Brüden aus Flashstufe
3: Sumpf aus Flashstufe
4: Kristaller-Umpump aus Umlaufkühler
5: Kristaller-Umpump in Umlaufkühler
6: Feststoff + Mutterlauge zu Filtration
9: Waschmittel
10: Mutterlauge + Waschlauge Rückführung
11: Feststoff (Produkt)
12: Purge zur Ausschleusung von Nebenkomponenten
13: Rückführung zur Reaktion
14: Produkt

Die folgenden Beispiele dienen der weiteren Erläuterung der Erfindung und sind nicht als Einschränkung zu sehen.

### Beispiele:

Verschiedene Mischungen von Diphenylcarbonat (DPC), Phenol, Pyridinhydrochlorid (Pyridiniumchlorid, im folgenden auch "Pyridin·HCl" bzw. "Py·HCl") und Salol (Phenylsalicylat, Nebenprodukt der Direktphosgenierung von Phenol) werden in einen mittels Doppelmantel temperierten Rührwerkskristallisator gegeben. Die Lösung wird langsam bis zum Beginn der Kristallisation auf 55 °C gekühlt. Anschließend wird die Temperatur konstant gehalten, bis die Kristallisation zum Erliegen gekommen ist. Das Gemisch wir auf einer temperierten Filternutsche abfiltriert. Die erhaltenen Kristalle werden mit reinem Phenol bei 50°C vermischt und anschließend abfiltriert. Dieser Waschvorgang wird wiederholt. Die Zusammensetzung der erhaltenen Kristalle wurde gaschromatographisch ermittelt.

### Beispiel 1: Phenol / DPC Verhältnis 1:1, 1,8 Gew.-% Pyridin-HCl:

| | Masse | Zusammensetzung Feed | Zusammensetzung Kristalle nach 2. Wäsche |
|---|---|---|---|
| | [g] | [Gew.-%] | [Gew.-%] |
| Phenol | 140,48 | 29,9 | 31,5 |
| PyHCl | 8,64 | 1,8 | 0,39 |
| DPC | 319,6 | 68,0 | 68,1 |
| Salol | 1,28 | 0,3 | 0,0 |
| **Summe** | **470** | | |

Bei Einsatz von einem Phenol / DPC Verhältnis von 1:1 kristallisiert wie erwartet im Wesentlichen das DPC/Phenol Addukt mit einem Massenverhätnis von ca. 70/30 aus. Der Katalysator PyHCl kann in diesem Versuch signifikant abgereichert werden.

### Beispiel 2: Phenol / DPC Verhältnis 0,68:1,4 Gew-% Pyridin·HCl:

| | Masse | Zusammensetzung Feed | Zusammensetzung Kristalle nach 2. Wäsche |
|---|---|---|---|
| | [g] | [Gew.-%] | [Gew.-%] |
| Phenol | 105 | 22,0 | 19,2 |
| PyHCl | 19,2 | 4,0 | 0,72 |
| DPC | 351,6 | 73,7 | 80,0 |
| Salol | 1,2 | 0,3 | 0,0 |
| **Summe** | **477** | | |

Bei Einsatz von einem Phenol / DPC Verhältnis von 0,68:1 kristallisiert im Wesentlichen reines DPC aus - das Massenverhältnis DPC/Phenol liegt bei ca. 80/20. Der Katalysator PyHCl kann in diesem Versuch ebenfalls signifikant abgereichert werden.

### Beispiel 3: Phenol / DPC Verhältnis 1,68:1,4 Gew-% Pyridin·HCl:

| | Masse | Zusammensetzung Feed | Zusammensetzung Kristalle nach 2. Wäsche |
|---|---|---|---|
| | [g] | [Gew.-%] | [Gew.-%] |
| Phenol | 260 | 41,1 | 34,0 |
| PyHcl | 19,2 | 3,0 | 0,02 |
| DPC | 351,6 | 55,6 | 66,0 |
| Salol | 1,2 | 0,2 | 0,0 |
| **Summe** | **632** | | |

Bei Einsatz von einem Phenol / DPC Verhältnis von 1,68:1 kristallisiert im Wesentlichen das das DPC/Phenol Addukt aus -das Massenverhältnis DPC/Phenol liegt bei ca. 80/20. Der Katalysator Py·HCl kann in diesem Versuch ebenfalls signifikant abgereichert werden.

Die Beispiele zeigen, dass unabhängig vom eingesetzten Verhältnis Phenol/DPC das Auskristallisieren von DPC bzw Phenol/DPC-Addukt möglich ist, wobei selbst im Labormaßstab eine fast vollständige Abreicherung des Katalysators in den Kristallen und dementsprechend eine Anreicherung in der Mutterlauge möglich ist.

Daher kann dieses Suspensionskristallisationsverfahren dazu eingesetzt werden, die Aufgabenstellung der Abtrennung des Pyridin-HCl Katalysators aus der Reaktionslösung, sowie dessen Rückführung in Verfahrensabschnitt I) zu erfüllen.

## Patentansprüche

1. Verfahren zur Herstellung von Diarylcarbonat durch Reaktion eines Monophenols mit Phosgen und/oder wenigstens einem Chlorameisensäurearylester in Gegenwart wenigstens eines gegebenenfalls substituierten Pyridins, in freier Form und/oder in Form seines Hydrochloridsalzes als Katalysator, **dadurch gekennzeichnet, dass**
a) die Reaktion in einem Reaktor unter Drücken von 1-50 bar durchgeführt wird,
b) das Reaktionsgemisch aus dem Reaktor in einen zur Suspensionskristallisation geeigneten Apparat überführt wird,
c) in dem Apparat durch Temperaturabsenkung die Suspensionskristallisation eingeleitet wird,
d) das entstandene Kristallisat von der zurückbleibenden katalysatorhaltigen Mutterlauge getrennt wird, und
e) die katalysatorhaltige Mutterlauge in den Reaktor des Schritts a) wenigstens teilweise zurückgeführt wird.

2. Verfahren nach Anspruch 1, **dadurch gekennzeichnet, dass** Phenol als Monophenol eingesetzt wird.

3. Verfahren nach Anspruch 1 oder 2, **dadurch gekennzeichnet, dass** höchstens 10 mol%, vorzugsweise höchstens 1 mol%, des gegebenenfalls substituierten Pyridins in freier Form vorliegen.

4. Verfahren nach Anspruch 3, **dadurch gekennzeichnet, dass** Pyridinhydrochlorid als Katalysator verwendet wird.

5. Verfahren nach Anspruch einem der Ansprüche 1 bis 4, **dadurch gekennzeichnet, dass** in keinem der Schritte a) bis e) eine wässrige Lösung eingesetzt wird.

6. Verfahren nach Anspruch einem der Ansprüche 1 bis 5, **dadurch gekennzeichnet, dass** der in Schritt a) entstehende Chlorwasserstoff nicht neutralisiert wird.

7. Verfahren nach Anspruch einem der Ansprüche 1 bis 6, **dadurch gekennzeichnet, dass** der in Schritt a) entstehende Chlorwasserstoff destillativ abgetrennt wird.

8. Verfahren nach einem der Ansprüche 1 bis 7, **dadurch gekennzeichnet, dass** das Verhältnis von Monophenol zu Diarylcarbonat vor dem Schritt c) mit Hilfe eines thermischen Trennverfahrens eingestellt wird.

9. Verfahren nach einem der Ansprüche 1 bis 8, **dadurch gekennzeichnet, dass** das Verhältnis von Monophenol zu Diarylcarbonat in Schritt c) mindestens 0,5 : 1, vorzugsweise mindestens 1 : 1, besonders bevorzugt mindestens 1,5 : 1 beträgt.

10. Verfahren nach einem der Ansprüche 1 bis 9, **dadurch gekennzeichnet, dass** in Schritt c) Impfkristalle eingesetzt werden.

11. Verfahren nach einem der Ansprüche 1 bis 10, **dadurch gekennzeichnet, dass** die Temperatur in Schritt c) auf unter 70 °C, vorzugsweise unter 60°C abgesenkt wird.

12. Verfahren nach einem der Ansprüche 1 bis 11, **dadurch gekennzeichnet, dass** das in Schritt d) abgetrennte Kristallisat mit Monophenol oder einem Monophenol/Diarylcarbonat-Gemisch gewaschen wird.

13. Verfahren nach einem der Ansprüche 1 bis 12, **dadurch gekennzeichnet, dass** das Kristallisat ein Addukt aus Monophenol und Diarylcarbonat umfasst.

14. Verfahren nach Anspruch 13, **dadurch gekennzeichnet, dass** aus den Adduktkristallen Diarylcarbonat und Monophenol gewonnen wird.

## Claims

1. Process for preparing diaryl carbonate by reaction of a monophenol with phosgene and/or at least one aryl chloroformate in the presence of at least one optionally substituted pyridine, in free form and/or in the form of the hydrochloride salt thereof, as catalyst, **characterized in that**
a) the reaction is carried out in a reactor under pressures of 1-50 bar,
b) the reaction mixture is transferred from the reactor into an apparatus which is suitable for the suspension crystallization,
c) in the apparatus, the suspension crystallization is introduced by lowering the temperature,
d) the resulting crystallizate is separated from the remaining catalyst-containing mother liquor, and
e) the catalyst-containing mother liquor is at least partly recirculated into the reactor of step a).

2. Process according to Claim 1, **characterized in that** phenol is used as monophenol.

3. Process according to Claim 1 or 2, **characterized in that** not more than 10 mol%, preferably not more than 1 mol%, of the optionally substituted pyridine is present in free form.

4. Process according to Claim 3, **characterized in that** pyridine hydrochloride is used as catalyst.

5. Process according to Claim any of Claims 1 to 4, **characterized in that** an aqueous solution is used in none of the steps a) to e).

6. Process according to Claim any of Claims 1 to 5, **characterized in that** the hydrogen chloride formed in step a) is not neutralized.

7. Process according to Claim any of Claims 1 to 6, **characterized in that** the hydrogen chloride formed in step a) is separated off by distillation.

8. Process according to any of Claims 1 to 7, **characterized in that** the ratio of monophenol to diaryl carbonate is set before step c) by means of a thermal separation process.

9. Process according to any of Claims 1 to 8, **characterized in that** the ratio of monophenol to diaryl carbonate in step c) is at least 0.5:1, preferably at least 1:1, particularly preferably at least 1.5:1.

10. Process according to any of Claims 1 to 9, **characterized in that** seed crystals are used in step c).

11. Process according to any of Claims 1 to 10, **characterized in that** the temperature is reduced to below 70°C, preferably below 60°C, in step c).

12. Process according to any of Claims 1 to 11, **characterized in that** the crystallizate separated off in step d) is washed with monophenol or a monophenol/diaryl carbonate mixture.

13. Process according to any of Claims 1 to 12, **characterized in that** the crystallizate comprises an adduct of monophenol and diaryl carbonate.

14. Process according to Claim 13, **characterized in that** the diaryl carbonate and monophenol is obtained from the adduct crystals.

## Revendications

1. Procédé de fabrication d'un carbonate de diaryle par réaction d'un monophénol avec du phosgène et/ou au moins un ester arylique de l'acide chloroformique en présence d'au moins une pyridine éventuellement substituée, sous forme libre et/ou sous la forme de son sel de chlorhydrate, en tant que catalyseur, **caractérisé en ce que**
a) la réaction est réalisée dans un réacteur à des pressions de 1 à 50 bar,
b) le mélange réactionnel est transféré depuis le réacteur dans un appareil approprié pour la cristallisation en suspension,
c) la cristallisation en suspension est amorcée dans l'appareil par abaissement de la température,
d) le cristallisat formé est séparé de la liqueur mère contenant le catalyseur restante, et
e) la liqueur mère contenant le catalyseur est au moins partiellement recyclée dans le réacteur de l'étape a).

2. Procédé selon la revendication 1, **caractérisé en ce que** du phénol est utilisé en tant que monophénol.

3. Procédé selon la revendication 1 ou 2, **caractérisé en ce qu'**au plus 10 % en moles, de préférence au plus 1 % en moles, de la pyridine éventuellement substituée se présente sous forme libre.

4. Procédé selon la revendication 3, **caractérisé en ce que** du chlorhydrate de pyridine est utilisé en tant que catalyseur.

5. Procédé selon la revendication l'une quelconque des revendications 1 à 4, **caractérisé en ce qu'**une solution aqueuse n'est utilisée dans aucune des étapes a) à e) .

6. Procédé selon la revendication l'une quelconque des revendications 1 à 5, **caractérisé en ce que** le chlorure d'hydrogène formé à l'étape a) n'est pas neutralisé.

7. Procédé selon la revendication l'une quelconque des revendications 1 à 6, **caractérisé en ce que** le chlorure d'hydrogène formé à l'étape a) est séparé par distillation.

8. Procédé selon l'une quelconque des revendications 1 à 7, **caractérisé en ce que** le rapport entre le monophénol et le carbonate de diaryle est ajusté à l'aide d'un procédé de séparation thermique avant l'étape c).

9. Procédé selon l'une quelconque des revendications 1 à 8, **caractérisé en ce que** le rapport entre le monophénol et le carbonate de diaryle à l'étape c) est d'au moins 0,5:1, de préférence d'au moins 1:1, de manière particulièrement préférée d'au moins 1,5:1.

10. Procédé selon l'une quelconque des revendications 1 à 9, **caractérisé en ce que** des cristaux d'inoculation sont utilisés à l'étape c).

11. Procédé selon l'une quelconque des revendications 1 à 10, **caractérisé en ce que** la température à l'étape c) est abaissée à moins de 70 °C, de préférence à moins de 60 °C.

12. Procédé selon l'une quelconque des revendications 1 à 11, **caractérisé en ce que** le cristallisat séparé à l'étape d) est lavé avec du monophénol ou un mélange de monophénol/carbonate de diaryle.

13. Procédé selon l'une quelconque des revendications 1 à 12, **caractérisé en ce que** le cristallisat comprend un adduit de monophénol et de carbonate de diaryle.

14. Procédé selon la revendication 13, **caractérisé en ce que** du carbonate de diaryle et du monophénol sont obtenus à partir des cristaux d'adduit.
